# EUROPÄISCHE PATENTSCHRIFT

(11) **EP 2 099 528 B1**
(45) Veröffentlichungstag und Bekanntmachung des Hinweises auf die Patenterteilung: **18.03.2015**
(21) Anmeldenummer: 07848041.5
(22) Anmeldetag: 11.12.2007
(51) Int. Cl.: A61K 8/26, A61K 8/28, A61K 8/39, A61K 8/44, A61K 8/894, A61K 8/04, A61K 8/06, A61K 8/34, A61K 8/58, A61Q 15/00, A61K 8/86

(54) **TRANSPARENTES ANTITRANSPIRANT-GEL**
TRANSPARENT ANTIPERSPIRANT GEL
GEL ANTITRANSPIRANT TRANSPARENT

(30) Priorität: 29.12.2006 DE 102006062564; 07.12.2007 DE 102007059297
(43) Veröffentlichungstag der Anmeldung: 16.09.2009
(73) Patentinhaber: Henkel AG & Co. KGaA, 40589 Düsseldorf (DE)
(72) Erfinder: BANOWSKI, Bernhard, 40597 Düsseldorf (DE); SCHMIDT, Melanie, 47802 Krefeld (DE); WADLE, Armin, 40699 Erkrath (DE)
(86) Internationale Anmeldenummer: PCT/EP2007/063651
(87) Internationale Veröffentlichungsnummer: WO 2008/080771

(56) Entgegenhaltungen:
- EP-A- 1 321 431
- EP-A- 1 491 182
- WO-A-00/69402
- WO-A-92/05767
- WO-A-99/33440
- WO-A-2006/013415
- DE-A1-102005 024 595
- US-A1- 2004 091 435
- US-A1- 2004 241 196

## Beschreibung

Die vorliegende Erfindung betrifft klare, schweißhemmende Gele in Form einer Wasser-in-Öl-Emulsion.

### Stand der Technik

Klare, schweißhemmende und desodorierende Gelzusammensetzungen sind beispielsweise in US 5587153 offenbart. Bei diesen Zusammensetzungen handelt es sich um Wasser-in-Öl-Emulsionen mit einer Viskosität von etwa 50 Pa·s bis 250 Pa·s (50.000 bis 250.000 cP) und bevorzugt etwa 100 bis 200 Pa·s (100.000 bis 200.000 cP). Die Wasserphase macht etwa 75 bis 90% der Zusammensetzung aus und enthält eine desodorierende oder schweißhemmende wirksame Menge (z. B. etwa 3 bis 25 Gew.-%) eines schweißhemmenden Wirkstoffes. Die Ölphase macht etwa 10 bis 25 Gew.-% der Zusammensetzung aus und enthält ein Siliconöl und einen Polyether-substituierten Siliconemulgator. Für optimale Klarheit muss der Brechungsindex der Ölphase und der Wasserphase innerhalb von etwa 0,001 oder besser und bevorzugt innerhalb von etwa 0,0004 aufeinander abgestimmt sein.
WO 96/06594 offenbart transparente schweißhemmende Wasser-in-Öl-Zusammensetzungen, die flüchtige Siliconöle und/oder flüchtige Kohlenwasserstofföle und siliconfreie Emulgatoren enthalten. EP 373424 offenbart transparente schweißhemmende Wasser-in-Öl-Zusammensetzungen, die Cyclotetrasiloxan und eine Mischung aus siliconfreien und siliconhaltigen Emulgatoren enthalten. WO 99/33440 A1 offenbart schweißhemmende Zusammensetzungen mit verringerter Rückstandsbildung nach Applikation auf die Haut, enthaltend 5 - 80 Gew.-% Cyclohexasiloxan, bezogen auf die gesamte Zusammensetzung, 0 - 35 Gew.-% Cyclotetrasiloxan und Cyclopentasiloxan, bezogen auf den Gesamtanteil an Cyclomethiconen in der Zusammensetzung und ein Siliconelatomer.
WO 00/69402 offenbart Antitranspirant- und Deodorant-Zusammensetzungen, die Cyclohexasiloxan zur Verbesserung der Produktstabilität, der Wirksamkeit und der kosmetischen Eigenschaften enthalten. Klare, schweisshemmende oder desodorierende Gelzusammensetzungen in Form einer Wasser-in-Öl-Emulsion werden in WO 00/69402 hergestellt.
Die vorstehend beschriebenen klaren schweißhemmenden und desodorierenden Gelzusammensetzungen haben den Nachteil der Fleckenbildung auf der Kleidung, die mit den Achselhöhlen des Anwenders in Kontakt gelangt. Dementsprechend sind große Anstrengungen unternommen worden, um das Anschmutzen von Gewebe zu verringern oder zu eliminieren, indem die Komponenten der Zusammensetzungen verändert wurden.
Ein weiteres Problem bei den bekannten Gelen ist ihr hoher Gehalt an Cyclomethicone. Bei den handelsüblichen Cyclomethiconen unterscheidet man vor allem Cyclotetrasiloxan, Cyclopentasiloxan und Cyclohexasiloxan. Cyclotetrasiloxan, dessen Schmelzpunkt mit -11°C ungewöhnlich hoch liegt, kann in den für ein Wasser-in-Öl-Emulsionsgel typischen höheren Einsatzmengen zu Problemen bei der Lagerstabilität führen. Cyclopentasiloxan ist eine relativ flüchtige Ölkomponente.
Aus diesem Grund wird es gern in Kosmetika, insbesondere in Antitranspirantien, eingesetzt, da es hilft, das Problem des Anschmutzens der Kleidung zu lösen. Andererseits bilden Antitranspirantien mit einem zu hohen Anteil an flüchtigem Cyclopentasiloxan auf der Haut weiße Rückstände, die schlecht auf der Haut haften und herunterrieseln können, was von vielen Verbrauchern als unangenehm empfunden wird.
Weiterhin wurde festgestellt, dass bestimmte Inhaltsstoffe, wie Siliconelastomere oder Öl-in-Wasser-Emulgatoren, nicht zu lagerstabil transparenten Gelzusammensetzungen führen.

### Aufgabenstellung

Eine Aufgabe der vorliegenden Erfindung war es, schweißhemmende oder desodorierende Gelzusammensetzung mit verringerter Rückstandsbildung ohne einen Verlust der Wirksamkeit oder ästhetischer Eigenschaften bereitzustellen.
Eine weitere Aufgabe der vorliegenden Erfindung war es, schweißhemmende oder desodorierende Gelzusammensetzungen bereitzustellen, die möglichst geringe Mengen an Cyclotetrasiloxan und Cyclopentasiloxan, bevorzugt weder Cyclotetrasiloxan noch Cyclopentasiloxan enthalten.
Eine weitere Aufgabe der vorliegenden Erfindung war es, klare, transparente schweißhemmende oder desodorierende Gelzusammensetzungen bereitzustellen.

Überraschend wurde gefunden, dass mit einem hohen Anteil an Cyclohexasiloxan als mittelflüchtiger bis flüchtiger Ölkomponente die nicht anfleckenden Eigenschaften der Zusammensetzung wesentlich verbessert werden und gleichzeitig eine hohe Lagerstabilität in Bezug auf die Viskosität und die Phasenseparation bei starken Temperaturwechseln erzielt werden konnte.

Gegenstand der vorliegenden Anmeldung ist eine klare schweißhemmende oder desodorierende Gelzusammensetzung in Form einer Wasser-in-Öl-Emulsion mit einer Viskosität im Bereich von 40 bis 250 Pa·s bei 21 °C, enthaltend 70 - 90 Gew.-% einer Wasserphase, darin gelöst 3% bis 25 Gew.-% mindestens eines schweißhemmenden Salzes und mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon, 10% bis 30 Gew.-% einer Ölphase, darin 5 - 25 Gew.-% Cyclohexasiloxan, einen Gesamtgehalt an Cyclotetrasiloxan und Cyclopentasiloxan von 0 bis maximal 5 Gew.-%, mindestens einen Polyether-substituierten Wasser-in-Öl-Siliconemulgator, ausgewählt aus Bis-PEG/PPG-14/14 Dimethicone und 0% bis 2 Gew.-% eines nichtflüchtigen Öls.

Die erfindungsgemäßen Gelzusammensetzungen stellen Wasser-in-Öl-Emulsionen dar und haben bevorzugt eine Viskosität im Bereich von 40 bis 250 Pa·s (40.000 bis 250.000 cP), bevorzugt 50 bis 150 Pa·s (50.000 bis 150.000 cP) und besonders bevorzugt 60 bis 100 Pa·s (60.000 bis 100.000 cP) bei 21°C.

Die Viskositätsangaben beziehen sich auf Messungen mit einem Rotationsviskosimeter der Firma Brookfield unter Auswahl der Spindel und der Umdrehungszahl, wie sie in dem Handbuch "More Solutions to Sticky Problems" der Firma Brookfield empfohlen ist:
bei Verwendung von T-Spindeln und Helipath:

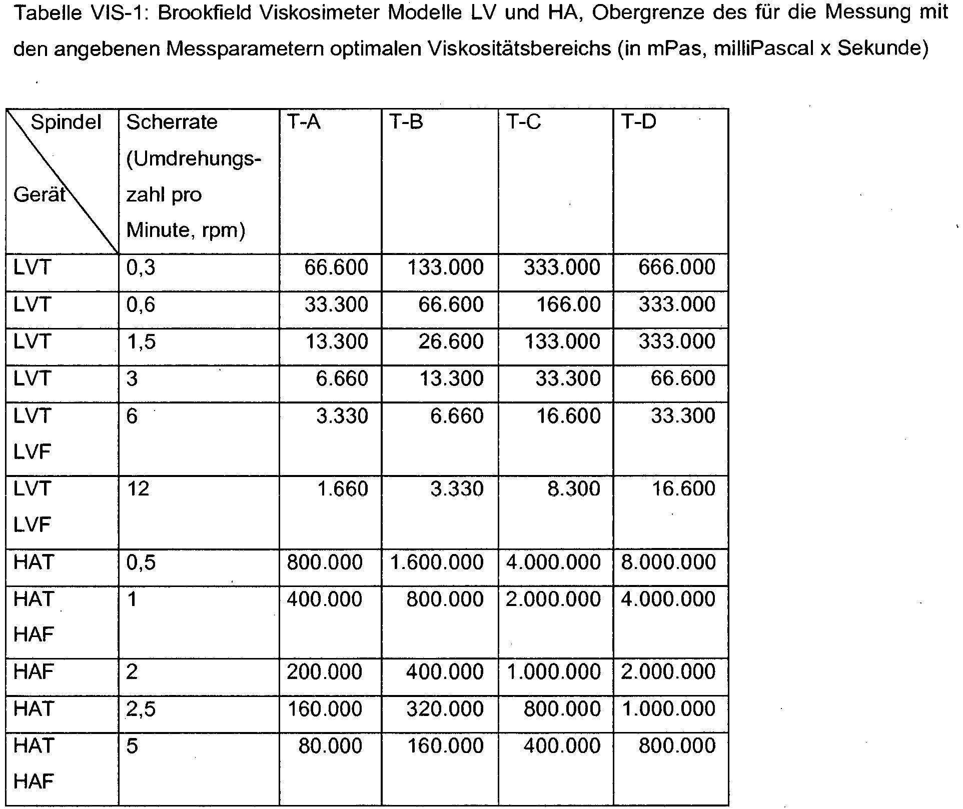

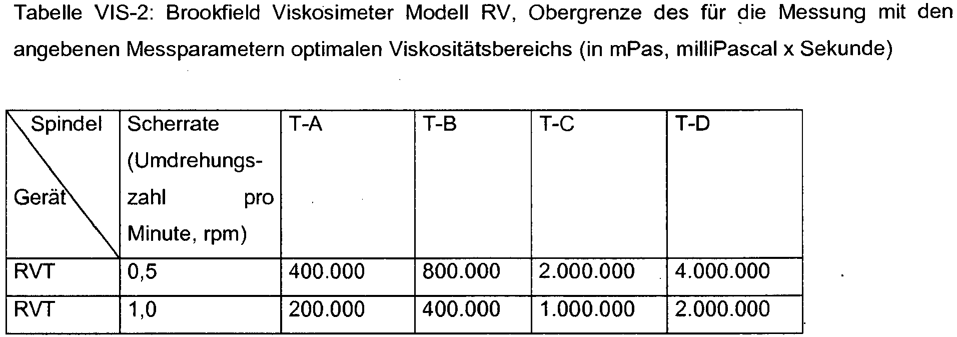

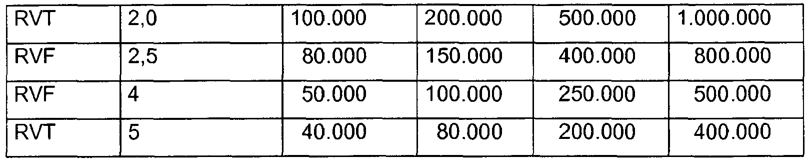

Die angegebene Viskosität stellt den oberen Grenzwert für den optimalen Messbereich für die jeweilige Spindel-Umdrehungszahl-Kombination dar. Falls für einen Viskositätsbereich zwei verschiedene Messparameter-Kombinationen möglich sind, wird die Spindel-Umdrehungszahl-Kombination gewählt, die den höheren Skalenteil-Wert liefert. Weiterhin beziehen sich die Viskositätsangaben auf die Zusammensetzung 24 Stunden nach Herstellung und bei einer Temperatur von 21 °C, wobei die Messungen unter Verwendung eines Helipaths durchgeführt werden.

Die Wasserphase macht 70 bis 90 Gew.-%, bevorzugt 75 bis 85 Gew.-% der erfindungsgemäßen Zusammensetzung aus und enthält eine desodorierende oder schweißhemmend wirksame Menge (3 bis 25 Gew.-%) eines darin aufgelösten schweißhemmenden Wirkstoffs. Die Ölphase macht 10 bis 30 Gew.-%, bevorzugt 15 bis 25 Gew.-% der erfindungsgemäßen Zusammensetzung aus. Der Gesamtgehalt an Cyclotetrasiloxan und Cyclopentasiloxan beträgt maximal 5 Gew.-%, bevorzugt 1 bis 3 Gew.-%, besonders bevorzugt 0,5 bis 1,5 Gew.-%, außerordentlich bevorzugt 0 Gew.-%, jeweils bezogen auf das Gewicht der erfindungsgemäßen Zusammensetzung.
Weiterhin enthalten die erfindungsgemäßen Gelzusammensetzungen einen Polyether-substituierten Wasser-in-Öl Siliconemulgator, ausgewählt aus Bis-PEG/PPG -14/14 Dimethicone de ebenfalls zur Ölphase gerechnet wird.
Bei den schweißhemmenden und desodorierenden Gelzusammensetzungen der vorliegenden Erfindung handelt es sich um Wasser-in-Öl-Emulsionen, in denen die Wasserphase 70 bis 90% der Zusammensetzung ausmacht. In der Wasserphase ist das schweißhemmende Salz gelöst, um einen schweißhemmenden oder desodorierenden Effekt zu erzielen.
Die erfindungsgemäßen Gelzusammensetzungen enthalten weiterhin mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit Xylitol, sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose sind erfindungsgemäß geeignet.
Besonders bevorzugte erfindungsgemäße Gelzusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit, Xylitol sowie Mischungen der vorgenannten Substanzen.
Die wasserlöslichen mehrwertigen C₂ - C₉-Alkanole mit 2 - 6 Hydroxylgruppen beziehungsweise die wasserlöslichen Polyethylenglycole mit 3 - 20 Ethylenoxid-Einheiten dienen vor allem dazu, den Brechungsindex der Wasserphase (n_{D} (Wasser) = 1,33) an den Wert der Ölphase (n_{D} (Öle) ≥ 1,38) anzugleichen. Je besser die Brechungsindices von Öl- und Wasserphase aneinander angeglichen werden, desto höher ist die Transparenz der erfindungsgemäßen Gelzusammensetzung. Bevorzugt werden die Brechungsindices von Öl- und Wasserphase innerhalb von ± 0,0004, bevorzugt innerhalb von ± 0,0003, aneinander angeglichen, um ein Maximum an Klarheit der fertigen Zusammensetzung zu erzielen. Die Gelzusammensetzung weist bevorzugt eine Klarheit von maximal 75 NTU (nephelometrische Trübungseinheiten) und besonders bevorzugt von maximal 50 NTU bei 21 °C auf.
Weiterhin verleihen die wasserlöslichen mehrwertigen C₂ - C₉-Alkanole mit 2 - 6 Hydroxylgruppen beziehungsweise die wasserlöslichen Polyethylenglycole mit 3 - 20 Ethylenoxid-Einheiten der Zusammensetzung eine hautpflegend-hydratisierende Wirkung. Außerdem tragen sie zur Maskierung von Rückständen der Gelzusammensetzung auf der Haut bei.

Bevorzugte erfindungsgemäße Gelzusammensetzungen sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten in einer Gesamtmenge von 10 - 50 Gew.-%, bevorzugt 15 - 30 Gew.-%, besonders bevorzugt 18 - 25 Gew.-%, außerordentlich bevorzugt 20 - 23 Gew.-%, jeweils bezogen auf die Gesamtzusammensetzung, enthalten ist.

Der Anteil des Wassers an der erfindungsgemäßen Gelzusammensetzung beträgt bevorzugt 20 - 60 Gew.-%, besonders bevorzugt 30 - 50 Gew.-%, außerordentlich bevorzugt 40 - 45 Gew.-%, jeweils bezogen auf die Gesamtzusammensetzung.

Antitranspirant-Wirkstoffe, die in den erfindungsgemäßen Gelzusammensetzungen verwendet werden können, schließen alle beliebigen konventionellen Aluminium-, Zirconium- und Aluminium-Zirconiumsalze ein, von denen bekannt ist, dass sie als schweißhemmende Wirkstoffe geeignet sind. Diese Salze schließen Aluminiumhalogenide und Aluminiumhydroxyhalogenide ein (z. B. Aluminiumchlorhydrat) sowie Mischungen und Komplexe davon mit Zirconyloxyhalogeniden und Zirconylhydroxyhalogeniden (z. B. Aluminium-Zirconiumchlorhydrat).
Bevorzugte schweißhemmende Wirkstoffe sind ausgewählt aus den wasserlöslichen adstringierenden anorganischen und organischen Salzen des Aluminiums, Zirkoniums und Zinks bzw. beliebigen Mischungen dieser Salze. Besonders bevorzugte Antitranspirant-Wirkstoffe sind ausgewählt aus den Aluminiumchlorhydraten, insbesondere den Aluminiumchlorhydraten mit der allgemeinen Formel [Al₂(OH)₅Cl 2-3 H₂O]ₙ, die in nicht-aktivierter oder in aktivierter (depolymerisierter) Form vorliegen können, weiterhin Aluminiumsesquichlorhydrat, Aluminiumchlorhydrex-Propylenglykol (PG) oder -Polyethylenglykol (PEG), Aluminium- oder Aluminiumzirkonium-Glycol-Komplexe, z. B. Aluminium- oder Aluminiumzirkonium-Propylenglycol-Komplexe, Aluminiumsesquichlorhydrex-PG oder -PEG, Aluminium-PG-dichlorhydrex oder Aluminium-PEGdichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-ZirkoniumChlorohydrat-Glycin-Komplexen wie Aluminiumzirconiumtrichlorhydrexglycin, Aluminiumzirconium-tetrachlorhydrexglycin, Aluminiumzirconiumpentachlorhydrexglycin, Aluminiumzirconiumoctachlor-hydrexglycin, Kaliumaluminiumsulfat (KAl(SO₄)₂ · 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexe von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat. Erfindungsgemäß wird unter Wasserlöslichkeit eine Löslichkeit von wenigstens 5 Gew.-% bei 20 °C verstanden, das heißt, dass Mengen von wenigstens 5 g des Antitranspirant-Wirkstoffes in 95 g Wasser bei 20 °C löslich sind.
In einer besonders bevorzugten Ausführungsform enthält die Zusammensetzung ein adstringierendes Aluminiumsalz, insbesondere Aluminiumchlorohydrat, das beispielsweise in Form einer wässrigen Lösung als Locron^{®} L von Clariant, als Chlorhydrol^{®} sowie in aktivierter Form als Reach^{®} 501 von Reheis vertrieben wird. Unter der Bezeichnung Reach^{®} 301 wird ein Aluminiumsesquichlorohydrat von Reheis angeboten, das ebenfalls besonders bevorzugt ist. Auch die Verwendung von Aluminium-Zirkonium-Tetrachlorohydrex-Glycin-Komplexen, die beispielsweise von Reheis unter der Bezeichnung Rezal^{®} 36G im Handel sind, kann erfindungsgemäß besonders bevorzugt sein.
Weitere bevorzugte Aluminiumsalze sind solche mit der allgemeinen Formel Al₂(OH)₆₋ₐXₐ, worin X Cl, Br, I oder NO₃ ist und "a" 0,3 bis 4 und bevorzugt 1 bis 2 beträgt, so dass das Molverhältnis von Al zu X im Bereich von 1 : 1 bis 2,1 : 1 liegt. In der Regel enthalten diese Salze Hydratationswasser, typischerweise in der Größenordnung von 1 bis 6 Mol Wasser pro Mol Salz. Am meisten bevorzugt ist das Aluminiumsalz Aluminiumchlorhydrat (d. h. X = Cl) mit "a" = 1, so dass das Molverhältnis von Aluminium zu Chlor 1,9 : 1 bis 2,1 : 1 beträgt.

Weitere bevorzugte Aluminium-Zirconiumsalze sind Mischungen oder Komplexe der vorstehend beschriebenen Aluminiumsalze mit Zirconiumsalzen der Formel ZrO(OH)_{2-pb}Y_{b}, worin Y Cl, Br, I, NO₃ oder SO₄ ist, "b" 0,8 bis 2 beträgt und "p" die Wertigkeit von Y ist. Auch die Zirconiumsalze enthalten in der Regel etwas Hydratationswasser, typischerweise in der Größenordnung von 1 bis 7 Mol Wasser pro Mol Salz. Vorzugsweise ist das Zirconiumsalz Zirconylhydroxychlorid der Formel ZrO(OH)_{2-b}Cl_{b}, worin "b" 1 bis 2 und bevorzugt 1,2 bis 1,9 beträgt. Die bevorzugten Aluminium-Zirconiumsalze haben ein Al : Zr-Verhältnis von 1,7 bis 12,5 und am meisten bevorzugt 2 bis 10, sowie ein Verhältnis von Metall/(X + Y) von 0,73 bis 2,1 und bevorzugt 0,9 bis 1,5. Ein bevorzugtes Salz ist Aluminium-Zirconiumchlorhydrat (d. h. X und Y sind Cl), das ein Al : Zr-Verhältnis von 2 bis 10 und ein Metall : Cl-Verhältnis von 0,9 - 2,1, bevorzugt 0,95 - 1,5, besonders bevorzugt 1 - 1,3, aufweist. Damit sollen in dem Begriff "Aluminium-Zirconiumchlorhydrat" die Formen Tri-, Tetra-, Penta- und Octachlorhydrat einbezogen sein. Die Aluminium-Zirconium-Salzkomplexe können außerdem eine neutrale Aminosäure enthalten, bevorzugt Glycin, typischerweise mit einem Gly : Zr-Verhältnis von ungefähr 1 : 1, d.h. 0,8- 1,2, besonders bevorzugt 1.

Die erfindungsgemäßen Gelzusammensetzungen sind dadurch gekennzeichnet, dass der mindestens eine Antitranspirant-Wirkstoff in einer Menge von 3 - 27 Gew.-%, vorzugsweise 5 - 22 Gew.-% und insbesondere 10 - 20 Gew.-%, enthalten ist, bezogen auf das Gesamtgewicht der Aktivsubstanz (USP, US Pharmacopoeia) in der Gesamtzusammensetzung.
Die in der vorliegenden Patentanmeldung in Gew.-% angegebene Menge an schweißhemmendem/n Salze/n ist nach der Methode der US Pharmacopoeia (USP) zu berechnen, nach der gebundenes Kristallwasser und andere Liganden, z. B. Glycin, ausgeschlossen sind. Bevorzugt werden die schweißhemmenden Salze in solubilisierter Form eingesetzt, d. h. gelöst in einem Lösemittel, bevorzugt gelöst in Wasser, Ethanol, Propanol, iso-Propanol, wasserlöslichen mehrwertigen C₂ - C₉-Alkanolen mit 2 - 6 Hydroxylgruppen (siehe oben), darunter bevorzugt 1,2-Propylenglycol, wasserlöslichen Polyethylenglycolen mit 3 - 20 Ethylenoxid-Einheiten (siehe oben), und Mischungen dieser Lösemittel, insbesondere Mischungen Wasser/Ethanol und Wasser/1,2-Propylenglycol. Bevorzugt werden schweißhemmende Salze als wässrige Lösungen, typischerweise mit einer Konzentration von 30 - 50 Gew.-%, eingesetzt. Außerordentlich bevorzugt werden solche Lösungen nicht durch erneutes Auflösen von sprühgetrockneten Salzen angesetzt, da sprühgetrocknete Salze Oxide enthalten, die eine Trübung in der fertigen Zusammensetzung hervorrufen können.

Die erfindungsgemäßen Gelzusammensetzungen liegen als Wasser-in-Öl-Emulsion vor und enthalten mindestens einen Polyether-substituierten Wasser-in-Öl-Siliconemulgator (W/O-Emulgator), ausgewählt aus Bis- PEG/PPE- 14/14 Dimethicone der die Emulgierung der Wasserphase in die Ölphase stabilisiert.
Bis-PEG/PPG-14/14 Dimethicone, ist in einer Mischung mit Cyclopentasiloxan im Gewichtsverhältnis 85 (Emulgator) : 15 (Cyclopentasiloxan) als Abil EM 97 (Goldschmidt) im Handel erhältlich.
Überraschend wurde festgestellt, dass der Wasser-in-Öl-Siliconemulgator Bis-PEG/PPG-14/14 Dimethicone eine schonende und energiesparende Emulsionsherstellung bei relativ niedr iger Scherrate bzw. niedrigem Scherenergieeintrag ermöglicht. Die Emulsionsherstellung mit PEG/PPG-18/18 Dimethicone/ Cyclomethicone-Lösungen hingegen verlangt einen hohen Scherenergieeintrag. Demzufolge ist der Wasser-in-Öl-Siliconemulgator Bis-PEG/PPG-14/14 Dimethicone erfindungsgemäß besonders bevorzugt.
Weitere erfindungsgemäß außerordentlich bevorzugte W/O-Emulgatoren sind Poly-(C₂-C₃)alkylen-glycol-modifizierte Silicone, die mit C₄-C₁₈-Alkylgruppen hydrophob modifiziert sind. Auch hier ergaben sich überraschende Vorteile in der Herstellung und Verarbeitung. Besonders bevorzugte W/O-Siliconemulgatoren sind Cetyl PEG/PPG-10/1 Dimethicone (früher: Cetyl Dimethicone Copolyol, erhältlich als Abil EM 90), Lauryl Dimethicone Copolyol, PEG-8 Cetyl Dimethicone, weiterhin Alkyl Methicone Copolyole und Alkyl Dimethicone Ethoxy Glucoside.

Erfindungsgemäß bevorzugte Gelzusammensetzungen enthalten mindestens einen W/O-Siliconemulgator in einer Gesamtmenge von 0,1 - 10 Gew.-%, bevorzugt 0,5 - 5 Gew.-%, besonders bevorzugt 1 - 3 Gew.-%, außerordentlich bevorzugt 1,2 - 2,1 Gew.-%, jeweils bezogen auf das Gesamtgewicht der erfindungsgemäßen Gelzusammensetzung.
Erfindungsgemäß besonders bevorzugte Gelzusammensetzungen enthalten als einzigen W/O-Siliconemulgator 2 - 3,5 Gew.-%, bevorzugt 2,1 - 3 Gew.-%, Bis-PEG/PPG-14/14 Dimethicone, bezogen auf das Gesamtgewicht der erfindungsgemäßen Gelzusammensetzung.

Die erfindungsgemäßen Gelzusammensetzungen enthalten bevorzugt mindestens 5 Gew.-% Ethanol, bezogen auf ihr Gesamtgewicht. Besonders bevorzugt ist ein Ethanolgehalt von 8 - 20 Gew.-%, außerordentlich bevorzugt 10 - 15 Gew.-% Ethanol. Überraschend wurde festgestellt, dass die Konfektionierung eines Ethanolgehalts als W/O-Emulsion das Ethanol wesentlich hautverträglicher macht. Dies wurde insbesondere beobachtet bei W/O-Emulsionen, die als einzigen W/O-Siliconemulgator Bis-PEG/PPG-14/14 Dimethicone
enthalten. Speziell mit Bis-PEG/PPG-14/14 Dimethicone wurde auch überraschend beobachtet, dass der Frischeeffekt, der durch den Ethanolgehalt der erfindungsgemäßen W/O-Emulsionen hervorgerufen wird, dem der herkömmlichen ethanolischen Lösungen entspricht beziehungsweise diesen sogar übertrifft, und zwar trotz des höheren Wassergehaltes.

In einer bevorzugten Ausführungsform sind die erfindungsgemäßen Gelzusammensetzungen frei von Siliconelastomeren.
Erfindungsgemäß versteht man unter Siliconelastomeren beispielsweise solche Siliconelastomere, die durch die Vernetzung eines Organopolysiloxans, das mit mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist, erhältlich sind.
Das Organopolysiloxan mit mindestens 2 C₂ - C₁₀ - Alkenyl-Gruppen mit terminaler Doppelbindung im Molekül ist ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropro-pyl)-polysiloxanen mit Dimethylvinylsiloxy-Endgruppen und Dimethylsiloxan-Methyl-(3,3,3-trifluoro-propyl)-siloxan-Copolymeren mit Dimethylvinylsiloxy-Endgruppen.
Das vernetzende Organopolysiloxan mit mindestens zwei Silicon-gebundenen Wasserstoffatomen ist ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogen-siloxan-Copolymeren.
Nicht-emulgierende Siliconelastomere sind im Handel erhältlich, beispielsweise von Dow Corning die Produkte DC 9040, DC 9041 oder DC 9509, von General Electric, z. B. die Handelsprodukt SFE 839 und GE 1229, von Shin-Etsu die Handelsprodukte KSG-15, KSG-16 oder KSG-18 sowie von Grant Industries die Produkte aus der Gransil^{®}-Serie, wie z. B. Gransil^{®}RPS Gel, INCI-Bezeichnung Cyclopentasiloxane and Polysilicone-11 oder Gransil^{®}GCM-4, INCI-Bezeichnung Cyclotetrasiloxane and Polysilicone-11.
Emulgierende Siliconelastomere enthalten als funktionelle Gruppen am Polysiloxan-Gerüst Polyoxyethylen- und/oder Polyoxypropylen-Gruppen. Diese Gruppen können endständig und/oder als Seitengruppen zur Polysiloxan-Kette angeordnet sein. Emulgierende Siliconelastomere sind im Handel erhältlich, beispielsweise von Shin-Etsu die Produkte KSG-21, KSG-31, KSG-31X, KSG-32 oder von Dow Corning das Handelsprodukt DC-9011.

In einer weiteren bevorzugten Ausführungsform sind die erfindungsgemäßen Gelzusammensetzungen frei von Öl-in-Wasser-Emulgatoren, insbesondere frei von Öl-in-Wasser-Emulgatoren mit einem HLB-Wert von 12 und darüber.

Die Ölphase macht 10 bis 30 Gew.-%, bevorzugt 15 - 25 Gew.-%, besonders bevorzugt 17 - 22 Gew.-% der erfindungsgemäßen Gelzusammensetzung aus, jeweils bezogen auf deren Gesamtgewicht. Die Ölphase stellt die äußere Phase dar und verleiht der Zusammensetzung ein besonders weiches, angenehmes Hautgefühl und - neben der schweißhemmenden eine pflegende kosmetische Wirkung.

Der Gehalt an Cyclohexasiloxan beträgt 5 - 25 Gew.-%, bevorzugt 8 - 20 Gew.-% und besonders bevorzugt 10 - 15 Gew.-%, bezogen auf das Gewicht der erfindungsgemäßen Gelzusammensetzung,

Der Gesamtgehalt an Cyclotetrasiloxan und Cyclopentasiloxan beträgt maximal 5 Gew.-%, bevorzugt 1 bis 3 Gew.-%, besonders bevorzugt 0,5 bis 1,5 Gew.-%, außerordentlich bevorzugt 0 Gew.-%, jeweils bezogen auf das Gewicht der erfindungsgemäßen Gelzusammensetzung.

Es wurde festgestellt, dass das Vorhandensein wesentlicher Mengen, das heißt, 2 Gew.-% und mehr, bezogen auf das Gesamtgewicht der erfindungsgemäßen Gelzusammensetzung, von nichtflüchtigen Ölen bewirkt, dass die Zusammensetzungen Bekleidungsstücke anflecken. Derartige nichtflüchtige Öle schließen nichtflüchtige Silicone ein, wie beispielsweise Polydimethylsiloxane (Dimethicone) mit einer kinematischen Viskosität (25 °C) von 1 × 10⁻⁵ m²/s (10 cSt) und mehr, sowie andere organische Öle als Emollient, wie insbesondere die Ester von linearen oder verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, beispielsweise Octylisononanoat. Derartige Öle werden normalerweise in die Zusammensetzung einbezogen, um ihr ein Geschmeidigmachen der Haut zu vermitteln und das Kleben oder die Klebrigkeit des Endproduktes zu verhindern. Bisher war es nicht möglich gewesen, die nichtflüchtige Ölkomponente der Gelzusammensetzung zu verringern, ohne die nachteilige Wirkung auf die ästhetischen Merkmale des Produktes hinzunehmen.
Bevorzugte erfindungsgemäße Gelzusammensetzungen sind frei von Kohlenwasserstoffölen, die Sauerstoffatome enthalten können, wie beispielsweise die vorgenannten Esteröle, und die keine Duftstoffe darstellen.

Weitere bevorzugte erfindungsgemäße Gelzusammensetzungen sind frei von Polydimethylsiloxanen (Dimethicone) mit einer kinematischen Viskosität (25 °C) von 1 × 10⁻⁵ m²/s (10 cSt) und mehr.

Bevorzugte erfindungsgemäße Gelzusammensetzungen enthalten mindestens ein flüchtiges lineares Silicon mit einer kinematischen Viskosität (25 °C) unter 1 × 10⁻⁵ m²/s (10 cSt).
Das flüchtige lineare Silicon dient bevorzugt dazu, einen Teil oder die gesamte nichtflüchtige Ölkomponente zu ersetzen. Dieses flüchtige lineare Silicon ist ein Polydimethylsiloxan oder Dimethicon, die eine verhältnismäßig geringe relative Molekülmasse haben, eine relativ geringe Viskosität und einen erheblichen Dampfdruck bei 25°C (d. h. ein Gramm der Flüssigkeit, die auf Filterpapier Nr. 1 aufgebracht wird, hinterlässt im Wesentlichen keinen sichtbaren Rückstand nach 30 min bei Raumtemperatur). Typischerweise hat sie außerdem einen Siedepunkt unterhalb von 250°C. Das flüchtige lineare Silicon (oder das flüchtige Dimethicone) werden dargestellt durch die Formel (CH₃)₃SiO(Si(CH₃)₂O)ₙSi(CH₃)₃, worin n eine ganze Zahl von Null bis 6 ist und bevorzugt 1 bis 4. Eine der Methyl-Gruppen der vorgenannten Formel lässt sich durch eine Alkyl-Gruppe ersetzen (z. B. mit 2 bis 10 Kohlenstoffatomen), um ein Alkylmethylsiloxan bereitzustellen. Ein solches Material schließt beispielsweise DC 2-1731 (Dow Corning) ein, bei dem es sich um 3-Hexylheptamethyltrisiloxan handelt (Viskosität 1 × 10⁻⁶ m²/s (1,0 cSt)). Obgleich ein reines Siliconpolymer zum Einsatz gelangen kann, ist das flüchtige lineare Silicon in der Regel eine Mischung von Siliconpolymeren der vorgenannten Formel. Das flüchtige lineare Silicon wird eine Viskosität unterhalb von 5 × 10⁻⁶ m²/s (5 cSt oder weniger als etwa 5 cP) haben und bevorzugt zwischen 0,6 × 10⁻⁶ und 3 × 10⁻⁶ m²/s (0,6 und 3,0 cSt) und mehr bevorzugt zwischen 1 × 10⁻⁶ und 2 × 10⁻⁶ m²/s (1,0 und 2,0 cSt). (Bei Siliconen mit einem spezifischen Gewicht bei 25°C im Bereich von 0,75 bis 0,92 gehen die vorgenannten Viskositätsbereiche über in 0,0005 bis 0,0028 Pa·s (0,5 bis 2,8 cP) und bevorzugt etwa 0,0008 bis 0,0018 Pa·s (0,8 bis 1,8 cP). Geeignete flüchtige lineare Silicone schließen Dimethicone mit 6,5 × 10⁻⁵ m²/s (0,65 cSt) (Hexamethyldisiloxan), Dimethicone 10⁻⁶ m²/s (1,0 cSt) (Octamethyltrisiloxan), Dimethicone 1,5 × 10⁻⁶ m²/s (1,5 cSt), Dimethicone 2,0 × 10⁻⁶ m²/s (2,0 cSt) (Dodecamethylpentasiloxan), DC 2-1184 und DC 2-1731 ein, die alle verfügbar sind bei Dow Corning. DC 2-1184, das eine Viskosität von etwa 1,7 × 10⁻⁶ m²/s (1,7 cSt) und eine mittlere relative Molekülmasse von etwa 320 hat (d. h. n beträgt etwa 1 bis 3 in der vorgenannten Formel) ist bevorzugt.
Die Menge des flüchtigen linearen Silicons, die in die Zusammensetzung einzuarbeiten ist, hängt von der Beschaffenheit des speziellen flüchtigen linearen Silicons ab, das zum Einsatz gelangt, sowie von den anderen Ölkomponenten, die in der Zusammensetzung vorhanden sind. Das bedeutet, man kann die Menge des flüchtigen linearen Silicons und die Menge des mittelflüchtigen bis flüchtigen Cyclohexasiloxans austarieren, um die gewünschte Ausgewogenheit von Fleckenfreiheit gegenüber Klebfreiheit oder das Geschmeidigmachen der Haut zu erzielen; das flüchtige lineare Silicon wird bevorzugt in einer Menge von 2 - 10 Gew.-% und besonders bevorzugt 3% bis 8 Gew.-%, bezogen auf das Gesamtgewicht der erfindungsgemäßen Gelzusammensetzung, eingesetzt.

Die erfindungsgemäßen Gelzusammensetzungen enthalten bevorzugt weiterhin mindestens eine Duftstoffkomponente. Als Duftstoffe oder Parfümöle können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Phenoxyethylisobutyrat, Benzylacetat, p-tert.-Butylcyclohexylacetat, Dimethylbenzylcarbinylacetat, Linalylacetat, Phenylethylacetat, Linalylbenzoat, Ethylmethylphenylglycinat, Benzylformiat, Allylcyclohexylpropionat, Styrallylpropionat und Benzylsalicylat. Zu den Ethern zählen zum Beispiel Benzylethylether, zu den Aldehyden z.B. die linearen Alkanale mit 8 bis 18 C-Atomen, Citral, Citronellal, Citronellyloxyacetaldehyd, Cyclamenaldehyd, Hydroxycitronellal, Lilial und Bourgeonal, zu den Ketonen z.B. die lonone α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Geeignete Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen- oder Ylang-Ylang-Öl. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Laudanumöl.
Die Duftstoffkomponente/n ist/sind bevorzugt in Mengen von 0,01 bis 4 Gew.-%, besonders bevorzugt 0,5 - 2 Gew.-%, jeweils bezogen auf das Gesamtgewicht der erfindungsgemäßen Gelzusammensetzung, enthalten.

Die erfindungsgemäßen Gelzusammensetzungen können vorteilhafterweise weiterhin mindestens einen hautkühlenden Wirkstoff enthalten. Erfindungsgemäß geeignete hautkühlende Wirkstoffe sind beispielsweise Menthol, Isopulegol sowie Mentholderivate, z. B. Menthyllactat, Menthylglycolat, Menthylpyrrolidoncarbonsäure, Menthylmethylether, Menthoxypropandiol, Menthonglycerinacetal (9-Methyl-6-(1-methylethyl)-1,4-dioxaspiro (4.5)decan-2-methanol), Monomenthylsuccinat und 2- Hydroxymethyl-3,5,5-trimethylcyclohexanol. Als hautkühlende Wirkstoffe bevorzugt sind Menthol, Isopulegol, Menthyllactat, Menthoxypropandiol und Menthylpyrrolidoncarbonsäure sowie Mischungen dieser Substanzen, insbesondere Mischungen von Menthol und Menthyllactat, Menthol, Mentholglycolat und Menthyllactat, Menthol und Menthoxypropandiol oder Menthol und Isopulegol.
Die erfindungsgemäßen Gelzusammensetzungen enthalten vorzugsweise mindestens einen hautkühlenden Wirkstoff in Mengen von 0,01 - 1 Gew.%, bevorzugt 0,02 - 0,5 Gew.% und besonders bevorzugt 0,05 - 0,2 Gew.%, jeweils bezogen auf das Gesamtgewicht der Gelzusammensetzung.
Obgleich die Zusammensetzungen der vorliegenden Erfindung mit den vorstehend beschriebenen Bestandteilen angesetzt und verwendet werden kann, kann es auch wünschenswert sein, andere wahlweise Komponenten zuzusetzen, um die angestrebten ästhetischen und andere Effekte zu erzielen. Beispielsweise kann es wünschenswert sein, antimikrobielle oder desodorierende Wirkstoffe, wie beispielsweise Triclosan, Konservierungsmittel und/oder Komplexbildner zuzusetzen.
Die Erfindung wird weiter mit Hilfe der folgenden Beispiele beschrieben, die lediglich zum Zwecke der Veranschaulichung dienen. Sämtliche Anteile und Gewichtsangaben sind auf das Gewicht der gesamten Gelzusammensetzung bezogen.

| | 1 Nicht Teil der Erfindung | 2 Nicht Teil der Erfindung |
|---|---|---|
| ALUMINUM ZIRCONIUM TETRACHLOROHYDREX GLY (50 %ige wässrige Lösung) | 6 | 23,5 |
| 1,2-Propylenglycol | 34,7 | 8,7 |
| Ethanol | - | 10 |
| DC 2-1184 (Dow Corning) | 3 | 4,8 |
| Cyclohexasiloxan, PEG/PPG-18/18 Dimethicone | 9 | 8,1 |
| DC 246 | 7 | 5 |
| Phenoxyethanol | 1,15 | - |
| Parfüm | 0,4 | 0,2 |
| Wasser | ad 100 | ad 100 |

DC 2-1184 (Dow Corning). Gemisch von linearen Polydimethylsiloxanen (mittlere Molmasse etwa 320 g/mol; Viskosität etwa 1,7 cSt)
DC 0,65 cSt, 200 Fluid (Dow Corning). Hexamethylendisiloxan (mittlere Molmasse etwa 162 g/mol; Viskosität etwa 0,65 cSt)
DC 246 (Dow Corning): mind. 95 Gew.-% Cyclohexasiloxan, max. 5 Gew.-% Cyclopentasiloxan

| | Nr. 3 | Nr. 4 | Nr. 5 | Nr. 6 | Nr. 7 |
|---|---|---|---|---|---|
| Abil EM 97 | 3,0 | 3,5 | 2,5 | 3,2 | 3,0 |
| Dow Corning 246 | 14,2 | 14,2 | 14,2 | 14,2 | 14,2 |
| Ethanol (96 %, DEP vergällt) | 10 | 5,3 | 10 | 8 | 10 |
| Parfüm/Duftstoff | 0,6 | 0,6 | 1,0 | 1,3 | 1,0 |
| Aluminiumhydroxychlorid (Aktivsubstanz, USP) | 20 | 20 | 22 | 18 | 19 |
| 1,2-Propylenglycol | 21,5 | 18 | 23 | 18 | 20 |
| Wasser | ad 100 | ad 100 | ad 100 | ad 100 | ad 100 |

Die vorgenannten Zusammensetzungen werden in der folgenden Weise hergestellt. Die Komponenten der wässrigen Phase und die Komponenten der Ölphase werden jeweils in separaten Behältern gemischt und gegebenenfalls filtriert und die jeweilige Brechzahl gemessen. Die Brechzahl der Wasserphase wird so eingestellt, dass sie der Brechzahl der Ölphase auf weniger als 0,0004 angepasst ist, indem nach Bedarf Wasser oder ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten, bevorzugt 1,2-Propylenglycol, zugesetzt werden. Die Wasserphase wird sodann langsam der Ölphase bei Raumtemperatur (z. B. 18°C) unter ausreichendem Mischen zur Erzeugung einer klaren Emulsion, gegebenenfalls mit minimaler Belüftung, zugesetzt. Diese Emulsion wird sodann unter Erzeugung eines klaren Gels mit einer Viskosität von etwa 40 bis 250 Pa·s (40.000 bis 250.000 cP) geschert.

## Patentansprüche

1. Klare, schweißhemmende oder desodorierende Gelzusammensetzung in Form einer Wasser-in-Öl-Emulsion mit einer Viskosität im Bereich von 40 bis 250 Pa·s bei 21°C, enthaltend
a) 70 - 90 Gew.-% einer Wasserphase, darin gelöst
a)i) 3% bis 25 Gew.-% mindestens eines schweißhemmenden Salzes und
a)ii) mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon,
b) 10% bis 30 Gew.-% einer Ölphase, darin
b)i) 5 - 25 Gew.-% Cyclohexasiloxan,
b)ii) einen Gesamtgehalt an Cyclotetrasiloxan und Cyclopentasiloxan von 0 bis maximal 5 Gew.-%,
b)iii) mindestens einen Polyether-substituierten Wasser-in-Öl-Siliconemulgator, ausgewählt aus Bis-PEG/PPG-14/14 Dimethicone,
b)iv) 0% bis 2 Gew.-% eines nichtflüchtigen Öls.

2. Gelzusammensetzung gemäß Anspruch 2, **gekennzeichnet durch** eine Viskosität im Bereich von 50 bis 150 Pa·s, besonders bevorzugt 60 bis 100 Pa·s bei 21 °C.

3. Gelzusammensetzung gemäß Anspruch 1 oder 2, **dadurch gekennzeichnet, dass** das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten in einer Gesamtmenge von 10 - 50 Gew.-%, bevorzugt 15 - 30 Gew.-%, besonders bevorzugt 18 - 25 Gew.-%, außerordentlich bevorzugt 20 - 23 Gew.-%, jeweils bezogen auf die Gesamtzusammensetzung, enthalten ist.

4. Gelzusammensetzung gemäß einem der vorstehenden Ansprüche, **dadurch gekennzeichnet, dass** mindestens 5 Gew.-% Ethanol, bevorzugt 8 - 20 Gew.-%, besonders bevorzugt 10 - 15 Gew.-%, jeweils bezogen auf die Gesamtzusammensetzung, enthalten ist.

5. Gelzusammensetzung gemäß einem der vorstehenden Ansprüche, **dadurch gekennzeichnet, dass** mindestens ein W/O-Siliconemulgator in einer Gesamtmenge von 0,1 - 10 Gew.-%, bevorzugt 0,5 - 5 Gew.-%, besonders bevorzugt 1 - 3 Gew.-%, außerordentlich bevorzugt 1,2 - 2,1 Gew.-%, jeweils bezogen auf das Gesamtgewicht der erfindungsgemäßen Gelzusammensetzung, enthalten ist.

6. Gelzusammensetzung gemäß einem der vorstehenden Ansprüche, **dadurch gekennzeichnet, dass** Bis-PEG/PPG-14/14 Dimethicone als einziger Wasser-in-Öl-Siliconemulgator und mindestens 5 Gew.-% Ethanol, bezogen auf die Gesamtzusammensetzung, enthalten sind.

7. Gelzusammensetzung gemäß einem der vorstehenden Ansprüche, **dadurch gekennzeichnet, dass** mindestens ein flüchtiges lineares Silicon enthalten ist.

8. Gelzusammensetzung gemäß einem der vorstehenden Ansprüche, **dadurch gekennzeichnet, dass** mindestens ein flüchtiges lineares Silicon in einer Gesamtmenge von 2 - 10 Gew.-% und bevorzugt 3% bis 8 Gew.-%, bezogen auf das Gesamtgewicht der erfindungsgemäßen Gelzusammensetzung, enthalten ist.

9. Gelzusammensetzung gemäß einem der vorstehenden Ansprüche, **gekennzeichnet durch** eine Klarheit von maximal 75 NTU (nephelometrische Trübungseinheiten) und besonders bevorzugt von maximal 50 NTU bei 21°C.

10. Gelzusammensetzung gemäß einem der vorstehenden Ansprüche, **dadurch gekennzeichnet, dass** 20 - 60 Gew.-%, bevorzugt 30 - 50 Gew.-%, besonders bevorzugt 40 - 45 Gew.-%, jeweils bezogen auf die Gesamtzusammensetzung, Wasser enthalten ist.

11. Gelzusammensetzung gemäß einem der vorstehenden Ansprüche, **dadurch gekennzeichnet, dass** sie frei von Siliconelastomeren ist.

12. Gelzusammensetzung gemäß einem der vorstehenden Ansprüche, **dadurch gekennzeichnet, dass** sie frei von Öl-in-Wasser-Emulgatoren, insbesondere frei von Öl-in-Wasser-Emulgatoren mit einem HLB-Wert von 12 und darüber, ist.

## Claims

1. Clear, antiperspirant or deodorant gel composition in the form of a water-in-oil emulsion having a viscosity in the range 40 to 250 Pa·s at 21 °C, comprising
a) 70 - 90 wt.% of an aqueous phase, in which is dissolved
a)i) 3% to 25 wt.% of at least one antiperspirant salt and
a)ii) at least one water-soluble polyhydric C₂ - C₉ alkanol containing 2 - 6 hydroxyl groups and/or at least one water-soluble polyethylene glycol containing 3 - 20 ethylene oxide units and mixtures thereof,
b) 10% to 30 wt.% of an oil phase, therein
b)i) 5 - 25 wt.% cyclohexasiloxane,
b)ii) a total content of cyclotetrasiloxane and cyclopentasiloxane of from 0 to maximum 5 wt.%,
b)iii) at least one polyether-substituted water-in-oil silicone emulsifier, selected from bis-PEG/PPG-14/14 Dimethicone,
b)iv) 0% to 2 wt.% of a non-volatile oil.

2. Gel composition according to claim 2, wherein the viscosity is in the range 50 to 150 Pa·s, particularly preferably 60 to 100 Pa·s at 21 °C.

3. Gel composition according to claim 1 or 2, comprising the at least one water-soluble polyhydric C₂ - C₉ alkanol containing 2 - 6 hydroxyl groups and/or at least one water-soluble polyethylene glycol having 3 - 20 ethylene oxide units in a total amount of 10 - 50 wt.%, preferably 15 - 30 wt.%, particularly preferably 18 - 25 wt.%, exceedingly preferably 20 - 23 wt.%, each based on the total composition.

4. Gel composition according to one of the preceding claims comprising at least 5 wt.% ethanol, preferably 8 - 20 wt.%, particularly preferably 10 - 15 wt.% water, each based on the total composition.

5. Gel composition according to one of the preceding claims, comprising at least one W/O silicone emulsifier in a total quantity of 0.1 to 10 wt.%, preferably 0.5 to 5 wt.%, particularly preferably 1 - 3 wt.% and exceendingly preferably 1.2 - 2.1 wt.%, each based on the total weight of the gel composition according to the invention.

6. Gel composition according to one of the preceding claims comprising bis-PEG/PPG-14/14 Dimethicone as the sole water-in-oil silicone emulsifier and at least 5 wt.% ethanol, based on the total composition.

7. Gel composition according to one of the previous claims comprising at least one volatile linear silicone.

8. Gel composition according to one of the preceding claims, comprising at least one volatile linear silicone in a total quantity of 2 to 10 wt.% and preferably 3% to 8 wt.%, based on the total weight of the gel composition according to the invention.

9. Gel composition according to one of the preceding claims, having a clarity of maximum 75 NTU (nephelometric turbidity units) and particularly preferably a maximum of 50 NTU at 21 °C.

10. Gel composition according to one of the preceding claims comprising 20 - 60 wt.%, preferably 30 - 50 wt.%, particularly preferably 40 - 45 wt.% water, each based on the total composition.

11. Gel composition according to one of the preceeding claims wherein said composition is exempt from silicone elastomers.

12. Gel composition according to one of the preceeding claims wherein said composition is exempt from oil-in-water emulsifiers, in particular exempt from oil-in-water emulsifiers with an HLB value of 12 and more.

## Revendications

1. Composition de gel claire désodorisante ou anti-transpirante se présentant sous la forme d'une agent émulsion eau-dans-huile ayant une viscosité dans la gamme de 40 à 250 Pa•s à 21 °C, ladite composition contenant
a) 70 à 90% en poids d'une phase aqueuse dans laquelle sont dissouts
a)i) 3% à 25% en poids d'au moins un sel anti-transpirant et
a)ii) au moins un alcanol en C₂-C₉ polyvalent hydrosoluble comportant 2 à 6 groupes hydroxyle et/ou au moins un polyéthylène-glycol hydrosoluble comportant 3 à 20 unités d'oxyde d'éthylène, et leurs mélanges,
b) 10% à 30% en poids d'une phase d'huile contenant
b)i) de 5 à 25% de cyclohexasiloxane,
b)ii) une teneur totale en cyclotétrasiloxane et cyclopentasiloxane de 0 à 5% maximum en poids,
b)iii) au moins un émulsifiant siliconé eau-dans-huile substitué avec du polyéther, choisie parmi les Bis-PEG/PPG-14/14-diméthicones,
b)iv) 0% à 2% en poids d'une huile non volatile.

2. Composition de gel selon la revendication 2, **caractérisée par** une viscosité dans la gamme de 50 à 150 Pa•s, notamment de préférence de 60 à 100 Pa•s à 21 °C.

3. Composition de gel selon la revendication 1 ou 2, **caractérisée en ce qu'**elle contient au moins un alcanol en C₂-C₉ polyvalent hydrosoluble comportant 2 à 6 groupes hydroxyle et/ou au moins un polyéthylène-glycol hydrosoluble comportant 3 à 20 unités d'oxyde d'éthylène dans une quantité totale de 10 à 50% en poids, de préférence 15 à 30% en poids, de façon particulièrement préférées de 18 à 25% en poids, encore plus préférablement de 20 à 23% en poids, rapportés à chaque fois à la composition totale.

4. Composition de gel selon l'une des revendications précédentes, **caractérisée en ce qu'**elle contient au moins 5% en poids d'éthanol, de préférence 8 à 20% en poids, de façon particulièrement préférée de 10 à 15% en poids, à chaque fois rapportés à la composition totale.

5. Composition de gel selon l'une des revendications précédentes, **caractérisée en ce qu'**elle contient au moins un émulsifiant siliconé eau-dans-huile dans une quantité totale de 0,1 à 10% en poids, de préférence de 0,5 à 5% en poids, de façon particulièrement préférée de 1 à 3% en poids, encore plus préférablement de 1,2 à 2,1% en poids à chaque fois rapportés au poids total de la composition de gel de l'invention.

6. Composition de gel selon l'une des revendications précédentes, **caractérisée en ce qu'**elle contient Bis-PEG/PPG-14/14-diméthicone comme unique émulsifiant siliconé eau-dans-huile et au moins 5% en poids d'éthanol rapportés à la composition totale.

7. Composition de gel selon l'une des revendications précédentes, **caractérisée en ce qu'**elle contient au moins une silicone linéaire volatile.

8. Composition de gel selon l'une des revendications précédentes, **caractérisée en ce qu'**elle contient au moins une silicone linéaire volatile dans une quantité totale de 2 à 10% en poids et de préférence de 3 à 8% en poids, rapportés au poids total de la composition de gel de l'invention.

9. Composition de gel selon l'une des revendications précédentes, **caractérisée par** une clarté de 75 UTN maximum (unités de turbidité néphélométrique) et de façon particulièrement préférée de 50 UTN maximum à 21 °C.

10. Composition de gel selon l'une des revendications précédentes, **caractérisée en ce qu'**elle contient de 20 à 60% en poids, de préférence de 30 à 50% en poids, de façon particulièrement préférée de 40 à 45% en poids, d'eau à chaque fois rapportés à la composition totale.

11. Composition de gel selon l'une des revendications précédentes, **caractérisée en ce qu'**elle est dépourvue d'élastomères de silicone.

12. Composition de gel selon l'une des revendications précédentes, **caractérisée en ce qu'**elle est dépourvue d'émulsifiants huile-dans-eau, notamment d'émulsifiants huile-dans-eau ayant un indice d'équilibre hydrophile/lipophile de 12 ou plus.
